# NEW EUROPEAN PATENT SPECIFICATION

(11) **EP 1 453 487 B2**
(45) Date of publication and mention of the opposition decision: **31.03.2021**
(45) Mention of the grant of the patent: 19.03.2008
(21) Application number: 01995903.0
(22) Date of filing: 23.11.2001
(51) Int. Cl.: A61K 9/28, A61K 9/48

(54) **PHARMACEUTICAL DOSAGE FORM WITH MULTIPLE COATINGS**
MEHRFACH BESCHICHETE PHARMAZEUTISCHE DOSIERUNGSFORM
FORME DE DOSE PHARMACEUTIQUE A FILMS MULTIPLES

(43) Date of publication of application: 08.09.2004
(73) Proprietor: Allergan Pharmaceuticals International Limited, Dublin 17 (IE)
(72) Inventor: DITTMAR, Gregory, Paul, Norwich, NY 13815 (US); AMANTE, Joseph, Michael, Norwich, NY 13815 (US); CRONK, Tony Ryan, Mishawaka, IN 46545 (US); NEWBY, Daniel, Gary, South New Berlin, NY 13843 (US)
(74) Representative: FRKelly
(86) International application number: PCT/US2001/043893
(87) International publication number: WO 2003/045356

(56) References cited:
- EP-A- 0 366 621
- EP-A- 0 453 001
- WO-A-98/27967

## Description

### TECHNICAL FIELD

The present invention relates to novel unit dosage forms comprising therapeutic agents with improved resistance to coating fractures during processing, manufacturing or packaging.

### BACKGROUND OF THE INVENTION

A number of prior art references teaches the advantages of delivery of therapeutic agents to the lower part of the gastrointestinal tract, especially the large intestine or the colon. These reference illustrate the difficulty of formulating dosage forms that will achieve this delivery benefit. For example, US Patent No. 5,541,170 and 5,541,171, Rhodes et al., both issued July 30, 1996, discuss the delivery of pharmacologically active agents, especially 5-aminosalicylic acid, to the large intestine for the treatment of colonic or rectal disorders. US Patent No. 5,686,105, Kelm et al., issued November 11, 1997, teaches colonic delivery of therapeutic agents wherein the dosage form comprises a coating system with at least one inner coating layer and one outer coating layer. The inner coating layer is an enteric polymer that begins to dissolve in an aqueous media at a pH between about 5 to about 6.3, and the outer coating layer is an enteric polymer that begins to dissolve in an aqueous media at a pH of between about 6.8 to 7.2. US Patent No. 5,171,580, Iamartino et al., issued Dec. 15, 1992, teaches pharmaceutical preparations containing an active ingredient to be released in the lower part of the gastrointestinal tract, the large intestine and especially the colon, consisting of a core with the active, the core being coated with three protective layers at different solubilities. This reference focuses on providing more specific and reliable release of a therapeutic active agent to the lower part of the gastrointestinal tract, especially the colon, achieved with the three protection layers, as well as the benefits of having a selective effect in the colon. Other prior art references also focus on the benefits of delivering therapeutic agents to the colon. These references include US Patent Nos. 5,686,106, Kelm et al., issued Nov. 11, 1997; 5,914,132, Kelm et al, issued June 22, 1999; 4,910,021, Davis et al, issued March 20, 1990; 4,432,966, Zeitoun et al., issued Feb. 21, 1984; 5,654,004, Okayama et al., issued August 5, 1997; 5,900,252, Calcanchi et al., issued May 4, 1999; 5,482,718, Shah et al, issued Jan. 9, 1996; 5,316,772, Jurgens et al., issued May 31, 1994; EP 225,189, Davies, et al, published June 10, 1987; and Khan et al., Drug Development and Industrial Pharmacy, 26(5), 549-554 (2000).

EP-A- 0 366 621, EP-A-0453001 and WO-A-9827967 disclose pharmaceutical dosage forms comprising multiple coatings.

None of the above prior art references, however, discusses the problem or possibility of coating fractures that may occur during processing, manufacturing, or packaging of the oral unit dosage form. Coating fractures may cause unreliable or inconsistent delivery or release of the therapeutic agent to the desired region of the gastrointestinal tract. These fractures may be associated with premature rupture or release of the unit dosage forms. Indeed, coating fractures may especially be problematic for larger than average size unit dosage forms or heavier unit dosage forms resulting from using larger dosages/levels of the therapeutic active.

The present invention, therefore, relates to solid unit dosage forms for oral administration in humans or lower animals which minimizes the impact or negative effects of coating fractures, especially for larger or heavier unit dosage forms. By reducing these negative effects, these compositions maintain the desired site of delivery of the therapeutic agents in the gastrointestinal tract.

### SUMMARY OF THE INVENTION

The present invention relates to a pharmaceutical composition in a solid unit dosage form for oral administration in a human or lower animal comprising:
a. a safe and effective amount of a therapeutically active agent, wherein the therapeutically active agent is 5-amino salicylic acid, at a dosage range of from 700 mg to 900 mg;
b. an inner coating layer, wherein the inner coating layer is poly(methacrylic acid, methyl methacrylate) 1:2; and
c. an outer coating layer comprising an enteric polymer applied onto the inner coating layer;
wherein the outer coating layer is a mixture of poly(methacrylic acid, methyl methacrylate) 1:1 and poly(methacrylic acid, methyl methacrylate) 1:2; wherein the inner coating layer and the outer coating layer contain no therapeutically active agent; and wherein the solid unity dosage form is a tablet.

### DETAILED DESCRIPTION OF THE INVENTION

The phrase "safe and effective amount", as used herein, means an amount of therapeutically active agent or other component of the present compositions, high enough to provide a significant positive modification of the condition to be treated, but low enough to avoid serious side effects (at a reasonable benefit/risk ratio), within the scope of sound medical judgment. A safe and effective amount of therapeutically active agent or other component of the present compositions, will vary with the particular condition being treated, the age and physical condition of the patient being treated, the severity of the condition, the duration of the treatment, the nature of concurrent therapy, the agent selected and like factors.

### Therapeutically Active Agent

The methods and compositions disclosed herein comprise a safe and effective amount of the therapeutically active agent. The therapeutic agents suitable for incorporation into dosage forms described here are those for which treatment of the colon is therapeutically advantageous. These include therapeutic agents useful for the treatment of diseases of the colon such as constipation, diarrhea, irritable bowel syndrome (IBS), Crohn's disease, colitis, ulcerative colitis, carcinomas, idiopathic protitis, infection in which systemic absorption of the therapeutic agent is neither required or desired, and other diseases or disorders of the colon or rectum. These include the actives 5-aminosalicylic compounds, 4-aminosalicylic compounds, sulfasalazine.

The therapeutically active agents are present in the solid dosage forms in suitable unit dosage amounts. These amounts will be known by those skilled in the art. In the pharmaceutical composition of the invention, the active agent is 5-amino salicylic acid at a dosage range of from 700mg to 900mg per tablet.

The therapeutically active agent may be incorporated into one of the several substrates described herein in a manner consistent with the physical chemical properties of the drug and its pharmacodynamics, using techniques known to those skilled in the art.

### The Inner and Outer Coating Layers

The coating layers of the present invention do not contain any therapeutically active agent of the present invention. In addition, the "coating layers" described herein refer to completely encasing or coating all of the solid unit dosage form (does not include coated microcrystal spheres, coated pellets, coated beads, coated microparticles or particles, or coated granules, of the therapeutically active agent).

### Inner Coating Layer

The inner coating layer is poly(methacrylic acid, methyl methacrylate) 1:2. Generally the inner coating layer is selected based on the preferred delivery site desired and is applied to the core of the unit dosage form to achieve a minimum coating thickness from 20 µm to 120 µm. The coating thickness depends on the actual size of the unit dosage form, but in one embodiment the minimum coating thickness of the inner coating layer is from 20 µm to 50 µm.

The inner coating layer is poly (methacrylic acid, methyl methacrylate) 1:2 (Eudragit^{®} S). Eudragit^{®} S, an anionic copolymer derived from methacrylic acid and methyl methacrylate, with a ratio of free carboxyl groups to the ester groups of approximately 1:2, and a mean molecular weight of approximately 135,000, from Rohm Tech.

### Outer Coating Layer

The outer coating layer comprises an enteric polymer applied onto the inner coating layer, wherein the inner coating layer is a mixture of poly(methacrylic acid, methyl methacrylate) 1:1 and poly(methacrylic acid, methyl methacrylate) 1:2. The outer coating material is a coating material that protects the inner coating layer from fractures during handling and that dissolves or is removed in the gastrointestinal tract prior to the inner coating layer. The outer coating layer is either a single coating or multiple coatings of the enteric polymer material. In another embodiment the unit dosage form has a single outer coating layer.

Generally the outer coating layer is applied to the core of the unit dosage form to achieve a minimum thickness of from 10 µm to 200 µm, in another embodiment is from 30 µm to 150 µm.

Thus, the outer coating layer is a mixture of poly(methacrylic acid, methyl methacrylate) 1:2 and poly(methacrylic acid, methyl methacrylate) 1:1.

In another embodiment the outer coating layer is a single layer of a mixture of poly(methacrylic acid, methyl methacrylate) 1:1 (Eudragit^{®} L) and poly(methacrylic acid, methyl methacrylate) 1:2 (Eudragit^{®} S) in a ratio of 1:10 to 1:10, preferably 1:5 to 1:3 more preferably 2:3. The coating thickness depends on the actual size of the unit dosage form, but in one embodiment the minimum coating thickness of the outer coating layer is from 10 µm to 50 µm, in another embodiment is from 20 µm to 40 µm.

In one embodiment, the function of the outer coating layer is to prevent or minimize fractures of the inner coating layer during formulation processing, manufacturing, and packaging, and the function of the inner coating layer is to maintain the desired point of release of the therapeutic active agent in the gastrointestinal tract. Since the inner coating is poly(methacrylic acid, methyl methacrylate) 1:2 (Eudragit^{®} S), the present invention maintains the desired point of release, as described, for example, in US Patent Nos. 5,541,170 and 5,541,171, Rhodes et al.

In one embodiment the total coating thickness (both the inner and outer coating layers together) is from 5 mg/cm² to 40 mg/cm², in another embodiment is from 10 mg/cm² to 15 mg/cm².

Eudragit^{®} L, is an anionic copolymer derived from methacrylic acid and methyl methacrylate, with a ratio of free carboxyl groups to the ester groups of approximately 1: 1, and a mean molecular weight of approximately 135,000, from Rohm Tech;
Eudragit^{®} L 30 D, is an aqueous acrylic resin dispersion, an anionic copolymer derived from methacrylic acid and ethyl acrylate with a ratio of free carboxyl groups to the ester groups of approximately 1:1, and a mean molecular weight of approximately 250,000; (it is supplied as an aqueous dispersion containing 30% w/w of dry lacquer substance);
Eudragit^{®} L 100-55, is an anionic copolymer derived from methacrylic acid and ethyl acrylate, with a ratio of free carboxyl groups to the ester groups of approximately 1: 1, and a mean molecular weight greater than about 100,000;

To enhance the elasticity of the coating materials, preferably the coating material of the present invention also comprises a plasticizer. Appropriate plasticizers include polyethylene glycols, propylene glycols, 1, 2-propylene glycol, dibutyl phthalate, diethyl phthalate, tributyl citrate, tributyrin, butyl phthalyl butyl glycolate (Santicizer^{®} B-16, from Monsanto, SL Louis, Missouri), triacetin, castor oil and citric acid esters; in another embodiment the plasitcizer is dibutyl phthalate, tributyl citrate, or triethyl citrate. These plasticizers are present in an amount to facilitate the coating process and to obtain an even coating film with enhanced physical stability. Generally the coating material comprises from 0% to 50% of a plasticizer, preferably from 2% to 25% by weight, more preferably from 10% to 20% by weight of the enteric polymer.

In addition, to facilitate the coating process, the coating material may also comprise inert solid particulates. Preferred inert solid particulates include talc and titanium dioxide.

The selections of optional plasticizer, optional inert solid particulate; and levels thereof, coating formulation type (solvent, ammoniated aqueous solution, or aqueous dispersion), and process are based upon the specific enteric polymer or film coatings used and the type of dosage form used according to criteria known to those skilled in the art. The solvent for the coating layers may be organic or aqueous. In one embodiment the coating layer is obtained via the use of an aqueous dispersion of the coating material.

### The Dosage Form and Method of Making the Dosage Form

A safe and effective amount of therapeutically active agent is incorporated into a solid unit dosage form. The term "solid unit dosage form" means any dosage form, preferably non-liquid, intended to be swallowed and having a sufficiently defined form to be coated. Solid unit dosage forms may be selected from the group consisting of a hard or soft capsule or a compressed tablet. The solid dosage form of the present invention is a tablet. Also described herein are the group consisting of soft gelatin capsules; hard gelatin capsules; and compressed tablets of any size or shape. In one embodiment the unit dosage form of the present invention comprises a unit dosage form from 550 mg to 1.5 gram total weight, in another embodiment from 600 mg to 1.2 grams total weight, and in even another embodiment from 750 mg to 1 gram total weight.

Described herein is a unit dosage form in the form of a spherical or elliptical soft elastic gelatin capsule. The soft elastic gelatin capsule is filled with therapeutically active agent suspended in a suitable vehicle compatible with the soft gelatin capsule.

Also described herein is a unit dosage form in the form of a hard capsule (i.e. starch or gelatin hard capsule), for example a starch capsule such as Capill^{®} from Cap- sulgel (Greenwood, SC) in which the length of the long axis of the capsule is less than 10 mm and not more than
1.5 times greater than the short axis diameter of the capsule. The capsule may be filled with a solid form of therapeutically active agent as described above, or alternatively with therapeutically active agent dissolved or suspended in a suitable vehicle compatible with the capsule wall.

In another embodiment the unit dosage form is a compressed spherical or elliptical tablet. The tablet is comprised of a solid form of therapeutically active agent and is compressed using conventional equipment and processes.

In addition to the therapeutically active agent the compositions of this invention also generally comprise pharmaceutiacally acceptable excipients. As used herein, "excipient" means one or more compatible solid or liquid filler diluents or encapsulating substances which are suitable for administration to a subject. The term "compatible", as used herein, means that the components of the composition are capable of being commingled with the active agent, and with each other, in a manner such that there is no interaction which would substantially reduce the pharmaceutical efficacy of the composition under ordinary use situations. Pharmaceutically-acceptable excipients must, of course, be of sufficiently high purity and sufficiently low toxicity to render them suitable for administration to the subject being treated. Excipients may act to facilitate incorporation of the therapeutically active agent into the dosage form, modify the release of the therapeutically active agent from the dosage form, stabilize the therapeutically active agent, or enhance absorption of the therapeutically active agent. Excipients should be safe for their intended use at the levels employed in the formulation. The formulation of therapeutically active agent and excipients is selected according to criteria well known to those skilled in the art to achieve the desired release rate, stability, absorption, and to facilitate the dosage form manufacture.

Some examples of pharmaceutically-acceptable excipients or components thereof are sugars, such as lactose, glucose, and sucrose; starches, such as cornstarch, potato starch, and sodium starch glycolate at a level of 1% to 8% by weight, in another embodiment from 2% to 4% by weight; cellulose and its derivatives, such as sodium carboxymethyl cellulose, ethyl cellulose, cel- lulose acetate; powdered tragacanth; malt; gelatin; talc; solid lubricants, such as stearic acid, magnesium stearate; or calcium sulfate; vegetable oils, such as peanut oil, cottonseed oil, sesame oil, olive oil, corn oil, and oil of theobroma; polyols such as propylene glycol, glycerin, sorbitol, mannitol, and polyethylene glycol; alginic acid; emulsifiers, such as the Tweens^{®}; wetting agents such as sodium lauryl sulfate; coloring agents; flavoring agents; excipients; tableting agents; stabilizers; antioxidants; preservatives; pyrogen-free water, isotonic saline; and phosphate buffer solutions. Excipients are described in Remington's Pharmaceutical Sciences, Mack Publishing Co. (19th edit. 1995); Modern Pharmaceutics, Vol. 7, Chapters 9 & 10, Banker & Rhodes (1979); Lieberman, et al, Pharmaceutical Dosage Forms: Tablets (1981); and Ansel, Introduction to Pharmaceutical Dosage Forms, 2d (1976). Their selection will depend on secondary considerations like taste, cost, and shelf stability, etc. which are not critical for the purposes of the subject invention, and can be made without difficulty by those skilled in the art.

In one embodiment all of the dosage forms of the present invention are uniform in size prior to coating with the coating layers. The uniform size allows for uniform coating thickness and more uniform dissolution of the coating layers.

Enteric polymers are generally applied onto the unit dosage forms as solutions in organic or aqueous solvents. The solvents commonly employed as vehicles are water, methylene chloride, ethanol, methanol, isopropyl alcohol, acetone, ethyl acetate and combinations thereof. The choice of the solvent is based primarily on the solubility of the polymer, ease of evaporation, and viscosity of the solution.

Some polymers are also available as aqueous systems. Unlike organic solutions, these aqueous-based systems can be prepared at high concentration without encountering high viscosity. Also, these aqueous systems do not have the problems associated with the organic systems such as flammability, toxicity of the residual solvent in the dosage form, etc.

Coating can be achieved by methods known to one skilled in the art such as by using fluidized bed equipment, perforated pans, a regular pharmaceutical pan, compression coating, continuous or short spray methods, or by drenching. For example, a plasticized dispersion of coating polymer may be applied onto the tablet core comprising the therapeutic active agent by spraying using any suitable spray equipment known in the art. In one embodiment the solid unit dosage forms are coated by continuous spray methods. In one embodiment the outer coating layer is applied after the inner coating layer but before the inner coating layer is dried and/or cured. In yet another embodiment the outer coating layer is applied immediately, e.g. within seconds, after the inner coating layer is applied. If a shiny finish coat is desired on the solid dosage forms of the present invention, a small quantity of polyethylene glycol can be applied to the finished dosage form.

The following non-limiting examples provide typical formulations for compositions of the present invention.

### Example 1

A wet granulation of 5-ASA (active ingredient), lactose, and povidone is blended with talc, magnesium stearate, sodium starch glycolate, and colloidal silicon dioxide. The blend is compressed into approximately 1034 mg tablets containing 800 mg of the active ingredient on a standard pharmaceutical rotary tablet press.

An inner layer of an EUDRAGIT ® S coating of 9.2 mg/cm² dried coating (i.e. about 62 microns) is applied to the core tablets first by pouring a portion of the coating formula without pigments and then by spraying coating onto the tablets. The coating suspension sprayed onto the tablets contains approximately 62% by weight on a dry basis of Eudragit ® S and is based in isopropyl alcohol and acetone with dibutylphthalate as the acting plasticizer.

An outer coating is either applied immediately following the application of the inner coating or once the inner coating has cured. The outer coating layer is sprayed onto the tablets to achieve of 4.1 mg/cm² dried coating (i.e. about 28 microns). This coating suspension contains approximately 61% by weight on a dry basis of EUDRAGIT ® S and L in a ratio of 3:2. It is based in isopropyl alcohol and acetone with dibutylphthalate as the acting plasticizer.

### Example 2 (not part of the claimed invention)

A wet granulation of 5-ASA (active ingredient), lactose, and povidone is blended with talc, magnesium stearate, sodium starch glycolate, and colloidal silicon dioxide. The blend is compressed into approximately 1570 mg tablets containing 1200 mg of the active ingredient on a standard pharmaceutical rotary tablet press.

An inner layer of an EUDRAGIT ® S and L mixture of 8.8 mg/cm² dried coating (i.e. about 60 microns) is applied to the core tablets first by pouring a portion of the coating formula without pigments and then by spraying coating onto the tablets. The coating suspension sprayed onto the tablets contains approximately 61 % by weight on a dry basis of Eudragit ® S and L in a ratio of 3:2 and is based in isopropyl alcohol and acetone with dibutylphthalate as the acting plasticizer.

An outer coating is applied immediately following the application of the inner coating or once the inner coating has cured. The outer coating layer is sprayed onto the tablets to achieve of 11.9 mg/cm² dried coating (i.e. about 80 microns). This coating suspension contains approximately 38% by weight on a dry basis of EUDRAGIT ® L and is based in isopropyl alcohol and acetone with triethyl citrate as the acting plasticizer.

## Claims

1. A pharmaceutical composition in a solid unit dosage form for oral administration in a human or lower animal comprising:
a. a safe and effective amount of a therapeutically active agent wherein the therapeutically active agent is 5-amino salicylic acid at a dosage range of from 700 mg to 900 mg;
b. an inner coating layer wherein the inner coating is poly(methacrylic acid, methyl methacrylate) 1:2 ; and
c. an outer coating layer comprising an enteric polymer applied onto the inner coating layer; wherein the outer coating layer is a mixture of poly(methacrylic acid, methyl methacrylate) 1:1 and poly (methacrylic acid, methyl methacrylate) 1:2; wherein the inner coating layer and the outer coating layer contain no therapeutically active agent: and wherein the solid unit dosage form is a tablet.

2. The composition of any of the preceding claims, wherein the total coating thickness of the inner and outer coating layers combined is from 5 mg/cm² to 40 mg/cm², preferably from 10 mg/cm² to 15 mg/cm².

3. The composition of any of the preceding claims wherein the solid dosage form is coated by continuous spray methods wherein the outer coating layer is applied after the inner coating layer but before the inner coating layer is dried or cured.

4. The composition of any of the preceding claims wherein the solid dosage form is a compressed tablet.

## Patentansprüche

1. Pharmazeutische Zusammensetzung in einer festen Einheitsdosierungsform für die orale Verabreichung an einen Menschen oder ein niederes Tier, die Folgendes umfasst:
a. eine sichere und wirksame Menge eines Therapeutikums, wobei das Therapeutikum 5-Aminosalicylsäure in einem Dosierungsbereich von 700 mg bis 900 mg ist;
b. eine Innenbeschichtungsschicht, wobei die Innenbeschichtung Poly(methacrylsäure-methylmethacrylat) 1 : 2 ist; und
c. eine Außenbeschichtungsschicht, die ein magensaftresistentes Polymer umfasst, das auf die Innenbeschichtungsschicht aufgetragen ist, wobei die Außenbeschichtungsschicht eine Mischung von Poly(methacrylsäuremethylmethacrylat) 1 : 1 und Poly(methacrylsäure-methylmethacrylat) 1 : 2 ist, wobei die Innenbeschichtungsschicht und die Außenbeschichtungsschicht kein Therapeutikum enthalten und wobei die feste Einheitsdosierungsform eine Tablette ist.

2. Zusammensetzung nach einem der vorstehenden Ansprüche, wobei die gesamte Beschichtungsdicke der Innen- und Außenbeschichtungsschicht zusammen von 5 mg/cm² bis 40 mg/cm², bevorzugt von 10 mg/cm² bis 15 mg/cm² beträgt.

3. Zusammensetzung nach einem der vorstehenden Ansprüche, wobei die feste Dosierungsform durch kontinuierliche Sprühverfahren beschichtet wird, wobei die Außenbeschichtungsschicht nach der Innenbeschichtungsschicht aufgetragen wird, doch bevor die Innenbeschichtungsschicht getrocknet oder ausgehärtet ist.

4. Zusammensetzung nach einem der vorstehenden Ansprüche, wobei die feste Dosierungsform eine gepresste Tablette ist.

## Revendications

1. Composition pharmaceutique sous une forme de dosage unitaire solide, destinée à une administration orale à un être humain ou un animal inférieur, comprenant :
a. une quantité sans danger et efficace d'un agent thérapeutiquement actif, l'agent thérapeutiquement actif étant l'acide 5-aminosalicylique, selon une plage de dosage allant de 700 mg à 900 mg ;
b. une couche de revêtement interne, le revêtement interne étant constitué de poly(acide méthacrylique, méthacrylate de méthyle) 1:2 ; et
c. une couche de revêtement externe comprenant un polymère entérique appliqué sur la couche de revêtement interne ; où la couche de revêtement externe est constituée d'un mélange de poly(acide méthacrylique, méthacrylate de méthyle) 1:1 et de poly(acide méthacrylique, méthacrylate de méthyle) 1:2 ; où la couche de revêtement interne et la couche de revêtement externe ne contiennent aucun agent thérapeutiquement actif, et où la forme de dosage unitaire solide est un comprimé.

2. Composition selon l'une quelconque des revendications précédentes, dans laquelle l'épaisseur de revêtement total des couches de revêtement interne et externe combinées va de 5 mg/cm² à 40 mg/cm², préférablement de 10 mg/cm² à 15 mg/cm².

3. Composition selon l'une quelconque des revendications précédentes, dans laquelle la forme de dosage solide est revêtue par des méthodes de pulvérisation continue où la couche de revêtement externe est appliquée après la couche de revêtement interne, mais avant que la couche de revêtement interne ne soit séchée ou durcie.

4. Composition selon l'une quelconque des revendications précédentes, dans laquelle la forme de dosage solide est un comprimé formé par compression.
